# EUROPEAN PATENT SPECIFICATION

(11) **EP 3 342 856 B1**
(45) Date of publication and mention of the grant of the patent: **04.05.2022**
(21) Application number: 17211074.4
(22) Date of filing: 29.12.2017
(51) Int. Cl.: C12N 5/00, C12N 5/078, A61K 35/14, A61K 35/16, A61K 35/19, A61K 35/15, A01N 1/02

(54) **PERIPHERAL BLOOD MONONUCLEATE CELLS FORMULATIONS SUBJECTED TO HYPOXIA**
ZUSAMMENSETZUNG AUS SAUERSTOFFMANGEL (HYPOXIE) BEHANDELTEN PERIPHERE MONONUKLEÄRE BLUTZELLEN
UNE COMPOSITION DES CELLULES MONONUCLÉES DE SANG PÉRIPHÉRIQUE TRAITÉES AVEC L'HYPOXIE

(30) Priority: 29.12.2016 IT 201600132406
(43) Date of publication of application: 04.07.2018
(73) Proprietor: Quantix Swiss SA, 7742 Poschiavo (CH)
(72) Inventor: COSTA, Massimo, 20121 Milano MI (IT); REHAK, Laura Olga Elena, 20121 Milano MI (IT); PANFILI, Graziano, 20121 Milano MI (IT)
(74) Representative: Perani & Partners S.p.A.

(56) References cited:
- WO-A1-2014/126931
- US-A1- 2013 121 979
- C. ROBERT VALERI: "Cryopreservation of Human Platelets and Bone Marrow and Peripheral Blood Totipotential Mononuclear Stem Cells", ANNALS OF THE NEW YORK ACADEMY OF SCIENCES, vol. 459, no. 1 Hematopoietic, 1 December 1985 (1985-12-01), pages 353-366, XP055399119, US ISSN: 0077-8923, DOI: 10.1111/j.1749-6632.1985.tb20845.x
- C R Valerl ET AL: "Effects of the temperature, the duration of frozen storage, and the freezing container on in vitro measurements in human peripheral blood mononuclear cells", , 1 April 1996 (1996-04-01), XP055399125, Retrieved from the Internet: URL:http://onlinelibrary.wiley.com/store/1 0.1046/j.1537-2995.1996.36496226141.x/asse t/j.1537-2995.1996.36496226141.x.pdf?v=1&t =j6f3oenu&s=bdfdeb43b8daf1052a830f82d4baa5 eee420aed1 [retrieved on 2017-08-16]
- A. SMAGUR ET AL: "Impact of different dimethyl sulphoxide concentrations on cell recovery, viability and clonogenic potential of cryopreserved peripheral blood hematopoietic stem and progenitor cells", VOX SANGUINIS., vol. 104, no. 3, 9 October 2012 (2012-10-09), pages 240-247, XP055223173, CH ISSN: 0042-9007, DOI: 10.1111/j.1423-0410.2012.01657.x
- LAUREN E. HIGDON ET AL: "Virtual Global Transplant Laboratory Standard Operating Procedures for Blood Collection, PBMC Isolation, and Storage :", TRANSPLANTATION DIRECT, vol. 2, no. 9, 1 September 2016 (2016-09-01), page e101, XP055398601, ISSN: 2373-8731, DOI: 10.1097/TXD.0000000000000613
- HARI RAMACHANDRAN ET AL: "Optimal Thawing of Cryopreserved Peripheral Blood Mononuclear Cells for Use in High-Throughput Human Immune Monitoring Studies", CELLS, vol. 1, no. 4, 25 July 2012 (2012-07-25), pages 313-324, XP055398624, DOI: 10.3390/cells1030313
- ÇIGDEM AKALIN AKKÖK ET AL: "Use of different DMSO concentrations for cryopreservation of autologous peripheral blood stem cell grafts does not have any major impact on levels of leukocyte-and platelet-derived soluble mediators", CYTOTHERAPY, vol. 11, no. 6, 1 January 2009 (2009-01-01), pages 749-760, XP055399091, GB ISSN: 1465-3249, DOI: 10.3109/14653240902980443

## Description

### FIELD OF THE INVENTION

The present invention describes an autologous preparation comprising peripheral blood mononuclear cells (PBMNCs), the autologous concentrate from which it is obtained and the production method thereof.

### DESCRIPTION

### BACKGROUND ART

The atherosclerotic disease of the lower limb arteries (PAD: Peripheral Arterial Disease) is caused by arteriosclerotic processes existing in the lower abdominal aorta, in the iliac, femoral, popliteal arteries which reduce the blood flow in the vessels of the legs (Dormandy JA, Ray S. The natural history of peripheral arterial disease. In: Tooke JE, Lowe GDO (Eds). Textbook of vascular medicine. London: Arnold, 1996:162-75).

The formation of arteriosclerotic plaque reduces (stenosis) the arterial lumen of the perfused vessels, up to arriving in some cases to the complete occlusion thereof, with consequent reduction or stopping of the blood flow in the involved district.

The symptoms resulting from stenosis or occlusion of the arterial lumen are extremely variable, depending on the degree of arterial obstruction and on the development of collateral arterial circulation in the lower limbs.

In relation to the severity thereof, PAD may therefore be in different clinical profiles, varying from the asymptomatic form, to the symptomatic condition (muscular weakness, *claudicatio intermittens*) up to critical limb ischemia (CLI), wherein part or all of the functionality thereof is put at risk by the pathological process under way.

Critical limb ischemia represents a syndrome which is associated to an unfavourable natural history with a result characterised by high mortality and morbidity rate (Golomb BA. Peripheral Arterial Disease: Morbidity and Mortality Implications. Circulation. 2006; 114:688-699; Diehm C, Allenberg JR, Pittrow D, Mahn M, Tepohl G, Haberl RL, Darius H, Burghaus I, Trampisch HJ, for the German Epidemiological Trial on Ankle Brachial Index Study Group. Mortality and Vascular Morbidity in Older Adults with Asymptomatic Versus Symptomatic Peripheral Artery Disease. Circulation. 2009; 120:2053-2061).

The patients most affected by this disease are diabetics, smokers, hypercholesterolemic, subjects wherein for different aetiology reasons arteriopathic processes are already under way. 25% of the patients undergoes amputation of the interested limb and 25% die within a year from the onset of the disease.

The incidence in Italy is about 15,000 patients, for 30% of which there are no therapeutic alternatives and amputation is indicated.

From 2001 to 2010 there were 11,639 patients that were subjected to amputation, of which 58.6% were diabetic (Official data Ministry of Health http://www.archeo.salute.gov.it/ricoveriOspedalieri/ricoveriOspedalieri.jsp).

The incidence of diabetes and of peripheral arterial disease (PAD) is increasing due to the ageing population (Savji N, Rockman CB, Skolnick AH, Guo Y, Adelman MA, Riles T, Berger JS. Association between advanced age and vascular disease in different arterial territories: a population database of over 3.6 million subjects. Journal of the American College of Cardiology. 2013; 61:1736-1743; Giorda CB, Avogaro A, Maggini M, Lombardo F, Mannucci E, Turco S, Alegiani SS, Raschetti R, Velussi M, Ferrannini E. Recurrence of Cardiovascular Events in Patients With Type 2 Diabetes. Diabetes Care. 2008; 31:2154-2159), with the majority of symptomatic patients over 60 years of age: around 0.6% in subjects of ages comprised between 45-54 years, 2.5% between 55-64 years, 8.8% between 65-74 years (Dormandy JA, Ray S. The natural history of peripheral arterial disease. In: Tooke JE, Lowe GDO (Eds). Textbook of vascular medicine. London: Arnold, 1996:162-75; Stoffers HE et al. Prevalence in general practice. In: Fowkes FGR. Epidemiology of peripheral vascular disease. London: Springer, 1991:109-15).

While surgical and/or intravascular therapy has reduced the incidence of amputations, the percentage of cuts in the critical limb ischemia remains high (25%), increasing in the light of the ageing population and of the incident increase of diabetes (Giorda CB, Avogaro A, Maggini M, Lombardo F, Mannucci E, Turco S, Alegiani SS, Raschetti R, Velussi M, Ferrannini E. Recurrence of Cardiovascular Events in Patients With Type 2 Diabetes. Diabetes Care. 2008; 31:2154-2159.)

It has been long known that patients affected by ischemic vascular diseases tend to spontaneously develop collateral vessels which avoid occlusion, thus guaranteeing the perfusion of the limb.

In recent years, great effort has been made in the development of innovative therapeutic approaches for stimulating the "self-healing" mechanisms of the organism, concentrating on the strategies for activating the growth of compensatory blood vessels which may help to stabilise the flow deficit, thus preserving tissue survival and organ function.

The autologous cell system at high angiogenic and vasculogenic capacity, falls within this scenario as a new therapeutic approach in the treatment of PAD and in particular in the cases of CLI.

The peripheral blood mononuclear cells, indicated in the scientific literature as PBMNCs, are represented by the monocytes/macrophage and the lymphocytes classes and constitute the autologous cellular substrate of excellence for the treatment of patients with critical limb ischemia not revascularisable.

The methodologies in use to date for producing concentrations containing PMMNCs provides that the treatment of biological material occurs in the operating room, wherein the autologous blood was drawn.

Optionally frozen as such to be stored over time, the blood of the patient is then concentrated in situ (hospital), where the selective filtration of the cellular material is done by the use of apheresis systems and/or through the use of medical-surgical aid, i.e. filters, centrifugation systems.

However, said selective filtration systems have limits that cannot be overcome, which affect the qualitative/quantitative composition of the final preparation, the treatment thereof and consequently the storage thereof over time.

In particular, the preparations obtained by the above-mentioned filtration systems are subject to:
- High neutrophil contamination, cellular components which significantly inhibit the PBMNC angiogenesis;
- Red blood cell contamination, the local inflammatory effect whereof makes the intramuscular administration less appreciated by the patient and may partially delay the anti-inflammatory effect of the cellular concentration;
- Little quantity of platelets, cellular subgroup which may, on the other hand, increase the angiogenic capacity of the peripheral mononuclear cells, thus working in synergy with PBMNCs.
- Resuspension of the concentration in saline solution where, on the other hand, a resuspension in autologous plasma could amplify the released angiogenic signals.

The above-mentioned content variations, besides making the qualitative/quantitative composition of the final concentration hard to standardise, alter the efficacy of the same, the biological activity thereof at the site of injection depends on the absolute and relative concentration of each present cellular subgroup.

The concentration thus obtained, must be administered to the patient in 6-8 hours subsequent to the treatment and may not be further stored, without there being alteration in terms of content and efficacy.

Furthermore, the resuspension after the administration, is normally done in saline solution rather than in plasma, choice which potentially reduces the local biological effect elicited by the cellular preparation.

For example, Valeri and colleagues (C.R. Valeri et al., Effects of the temperatures, the duration of frozen storage, and the freezing container on in vitro measurements in human peripheral blood mononuclear cells, TRANSFUSION, Vol. 36, No. 4, 1996, pages 303-308) describes a study that aims evaluating the influence of temperature, duration of frozen storage and type of cryopreservative container on the viability of peripheral blood mononuclear cells (PMBNCs). According to the study, the PBMNCs are isolated with Ficoll-hypaque density centrifugation procedure, from the buffy coat obtained during plateletpheresis of blood samples; the PBMNCs are treated with DMSO to obtain a final DMSO concentration of 10%; each unit is divided in 6 portions and collected in containers of different type; each portion is frozen at -80°C (2-4C°/min) and preserved at -80°C, -135°C or -150°C according to the type of container used. Once frozen, the samples are washed with saline solutions and centrifuged; the supernatant is discarded and the PBMNCs are then suspended in the autologous plasma obtained from patients. The author believes indeed that post-thaw washing reduces the risk of toxic effects and appears to improve *in vivo* survival (C. Robert Valeri Cryopreservation of Human Platelets and Bone Marrow and Peripheral Blood Totipotential Mononuclear Stem Cells, ANNALS OF NEW YORK ACADEMY OF SCIENCES, Vol 459, no.1 Hematopoietic, 1 December 1985, pages 353-366).

Recent studies in vitro have highlighted how hypoxic treatments may induce the cells to express the angiogenic capacity thereof, representing a possibility of improvement in the treatment efficacy of cellular ischemic tissue therapy products (Hypoxia-based strategies for angiogenic induction - The dawn of a new era for ischemia therapy and tissue regeneration - Organogenesis 9:4, 261-272; October/November/December 2013; ^{©} 2013 Landes Bioscience).

### SUMMARY OF THE INVENTION

The Applicant has found that it is possible to overcome the above-mentioned drawbacks through the autologous preparation of the present invention (A), substantially free of red blood cells and neutrophil-depleted, comprising mononuclear cells suspended in autologous platelet-enriched plasma (B) or optionally in platelet concentrate (B').

An object of the present invention is an autologous preparation (A), obtained at the moment of use for suitable dilution of a concentrate (C), in autologous platelet-enriched plasma (B) or in platelet concentrate (B').

The concentrate (C) is prepared under laminar flow hood A in a sterile environment B within a cell factory authorised for the production of advanced therapy products for pharmaceutical experiments, with a method for controlled, standardised separation, which responds to quality requirements defined by the GMP regulations in force in the field of Advanced Therapies Medicinal Products (ATMP).

### DESCRIPTION OF THE FIGURES

**Figure 1****:** Measurement of the cellular viability of thawed autologous concentrate, prepared with DMSO at 3% and at 10%. The measurements have been made at the moment of thawing (t = 0), after 1, 7, 30 and 45 days from the thawing (t = 1, t = 7, t = 30, t = 45).
**Figure 2****:** Measurement of the cellular yield of thawed autologous concentrate, prepared with DMSO at 3% and at 10%. The measurements have been made at the moment of thawing (t = 0), after 1, 7, 30 and 45 days from the thawing (t = 1, t = 7, t = 30, t = 45).
**Figure 3****:** Measurement of the cellular viability of thawed autologous concentrate, prepared with DMSO at 10 and at 5% and human albumin in concentrations equal to 200 g/l.
**Figure 4****:** Measurement of the cellular viability of thawed autologous concentrate, prepared with DMSO at 5% and human albumin at 5%. The measurements have been made at the moment of thawing (t = 0), and after 7 days from the thawing, on the following samples:
   - DMSO plasma 10% albumin 5%
   - DMSO plasma 5 % albumin 5%
   - Saline solution / plasma 50% DMSO 10%
   - Saline solution/ plasma 50% DMSO 5%
**Figure 5****:** Characterisation in flow cytometry of the cellular subpopulations. T lymphocytes (T linfo); B lymphocytes (B linfo); Monocytes (Mono); Neutrophils (Neutro/Granulo); Natural Killer Lymphocytes (NK).
**Figure 6****:** VEGF mRNA hypoxia at 1 h in fresh culture (ctl fresh), after hypoxia in fresh culture (ipox fresh), frozen control (ctl cong) and frozen hypoxia (ipox cong)

### DETAILED DESCRIPTION OF THE INVENTION

The object of the present invention is an autologous preparation (A) substantially free of red blood cells and neutrophil-depleted, comprising:
a) peripheral blood mononuclear cells (PBMNCs),
b) Dimethylsulfoxide (DMSO) in concentrations comprised between 0.3% and 1.3% (v/v),
c) autologous platelet-enriched plasma (B), having a platelet concentration comprised between 700,000 platelets/µL and 1,200,000 platelets/µL; or platelet concentrate (B'), having a platelet concentration comprised between 7,000,000 platelets/µL and 12,000,000 platelets/µL,
   said autologous preparation (A) being obtained with a process comprising the following steps:
   i) Obtaining, by treating a blood sample taken from the patient, autologous PBMNCs and platelet-enriched plasma (B) or platelet concentrate (B'), respectively; 15
   ii) Adding DMSO to the PBMNCs obtained at step (i), at a concentration comprised between 3% and 5% (v/v) dissolved in a portion of platelet-enriched plasma (B) or platelet concentrate (B') prepared in step i) and obtaining an autologous concentrate (C);
   iii) Freezing the autologous concentrate (C) of item ii) and the autologous 20 platelet-enriched plasma (B) or platelet concentrate (B') as obtained at step i);
   iv) Sending the autologous concentrate (C) and the autologous platelet-enriched plasma (B) or platelet concentrate (B') of step iii) to the desired health care centre; 25
   v) Thawing, upon use, the autologous concentrate (C) and the autologous platelet-enriched plasma (B) or platelet concentrate (B') of item iv),
   vi) Diluting, 7 to 10 times, the volume of thawed autologous concentrate (C) with thawed autologous plasma (B) or thawed platelet concentrate (B') and obtaining the autologous preparation (A),
      wherein said autologous concentrate (C) comprises mononuclear cells (PBMNCs), is red blood cell-free, neutrophil-depleted and comprises dimethylsulfoxide (DMSO) at a concentration comprised between 3vol% and 5vol% based on the volume of autologous concentrate (C).

For the objects of the present invention, autologous preparation (A) means a cellular product, for therapeutic use, wherein the cells intended for eliciting the biological effect are taken from the blood of the patient, processed outside of the organism and finally reintroduced in the original patient for the treatment of a disease.

In the objects of the present invention, peripheral blood mononuclear cells (PMNCs) means all the circulating blood cells, histologically characterised by a round nucleus and comprising monocytes, lymphocytes, mononuclear cells (MNCs) and the total of nucleated cells.

The autologous preparation (A) of the present invention is substantially free of red blood cells, where substantially free of red blood cells means a preparation wherein the red blood cell content is below the detectable limit.

Preferably the autologous preparation (A) is free of red blood cells, where free means that the red blood cell concentration is lower than 0.01%.

For the objects of the present invention, neutrophil-depleted autologous preparation (A) means an autologous preparation wherein the number of neutrophils is lower than 0.030 × 10⁸.

Even more preferably, the autologous preparation (A) contains a neutrophil content comprised between 0.015 × 10⁸ and 0.029 × 10⁸ (75 and 145 cells/µl).

For the objects of the present invention, the term autologous platelet-enriched plasma (B) means the liquid fraction of the blood, free of corpuscular fraction comprising the PBMNCs and free of red blood cells, through a process of treating a whole blood sample.

It should be noted that the autologous platelet-enriched plasma (B) is obtained by treating a whole blood sample taken from the patient, processed outside of the organism, and finally reintroduced in the original patient for the treatment of a disease.

In other words, said autologous preparation (A) and said platelet-enriched autologous concentrate (B) are obtained from one same patient.

For the objects of the present invention, platelet concentrate (B') means the heaviest liquid phase coming from the centrifugation of the autologous platelet-enriched plasma (B).

It should be noted that the platelet concentrate (B') is also autologous, according to the previously given definitions, since it is obtained through centrifugation of the autologous platelet-enriched plasma (B).

According to an embodiment of the invention, the autologous preparation (A) contains the autologous platelet-enriched plasma (B), where the platelet concentration in said autologous platelet-enriched plasma (B) is comprised between 700,000 platelets/µL and 1,200,000 platelets/µL.

Alternatively, the autologous preparation (A) contains the platelet concentrate (B'), where the platelet concentration in said platelet concentrate (B') is comprised between 7,000,000 platelets/µL and 12,000,000 platelets/µL.

The autologous platelet-enriched plasma (B) or the platelet concentrate (B') already comprise growth factors involved in the tissue regeneration processes and in the angiogenic processes, among which the vascular endothelial growth factor (VEGF), the fibroblast growth factor (FGF), the platelet-derived growth factor (PDGF), TGF-α e TGF-β.

The autologous preparation system of the present invention, by virtue of the high platelet concentration, increases the VEGF expression, physiologically produced by the smooth muscular cells and by those endothelial, configuring a paracrine signalling system, wherein the VEGF producing smooth muscular cells, and the endothelial cells, located near the former and these also VEGF producers, are target of the vascular endothelial growth factor.

The main effects of the VEGF consist in the induction of the proliferation and migration of endothelial cells, in the increase of capillary permeability, in the induction of the release of nitroxide and in the induction of the enzyme expression such as the serine proteases and the interstitial collagenases which have an important role in mediating interstitial remodelling phenomena which always accompany vascular neoformation processes.

The biological effect of the FGF-2 comprises an intense mitogenic activity against the endothelial cells and the smooth vascular muscular cells.

According to a preferred embodiment, the autologous preparation (A) contains albumin.

Said albumin is preferably human serum albumin.

In the autologous preparation (A) of the present invention, the peripheral blood mononuclear cells (PBMNCs) preferably comprise:
- Total nucleated cells (TNCs) comprised between 1.26 × 10⁸ (6.3 × 10³ cells/µl) and 1.89 × 10⁸ (9.45 × 10³ cells/µl),
- Lymphocytes comprised between 0.97 × 10⁸ (4.85 × 10³ cells/µl),) and 1.45 × 10⁸ (7.25 × 10³ cells/µl),
- Monocytes comprised between 0.28 × 10⁸ (1.4 × 10³ cells/µl),) and 0.42 × 10⁸ (2.1 × 10³ cells/µl),
- Mononuclear cells (MNCs) comprised between 1.25 × 10⁸ (6.25 × 10³ cells/µl) and 1.87 × 10⁸ (9.35 × 10³ cells/µl).

The set of peripheral blood mononuclear cells present in the autologous preparation of the present invention, in combination with the autologous platelet-enriched plasma (B), or the platelet concentrate (B') participate to the amplification of the local paracrine effect, and exhibit a synergy effect both in the angiogenic and anti-inflammatory activity.

Recent scientific studies (Tateno K et al Critical Roles of Muscle-Secreted Angiogenic Factors in Therapeutic Neovascularization Circulation Research Volume 98(9):1194-1202 May 12, 200) seem to suggest that the messenger most involved in the paracrine stimulation of the peripheral blood mononuclear cells is the IL-1β cytokine (1β Interleukin).

The set of peripheral blood mononuclear cells stimulates tissue regeneration and, through the increased expression of IL-1β by the regenerated myocytes, promotes the neovascularisation by induction of the expression of angiogenic factors, such as for example MCP-1 (monocyte chemoattractant protein-1) and VEGF.

Monocytes and platelets increase the release of cytokine and cellular adhesion molecules (selectin, ICAM-1, VCAM-1), responsible in turn for the expression of the VEGF and of the FGF, as well as for other vascular growth factors.

The use of autologous platelet-enriched plasma (B) or platelet concentrate (B') as suspension means for the peripheral blood mononuclear cells, not only has the great advantage of recycling biological material which would otherwise be lost, but also allows to resuspend the mononuclear fraction of the blood in an active liquid component, rich in growth factors, absent in the saline solutions typically used in the prior art.

The use of autologous platelet-enriched plasma (B) or platelet concentrate (B') therefore allows the storage of blood autocrine signalling systems, which would be lost by depletion of the plasma and platelet fraction, moreover responsible for the amplification of the biological effect elicited by the autologous preparation of the present invention, along with the selective concentration of blood components.

The autologous preparation (A) of the present invention is intended for use as a medicament.

The autologous preparation (A) of the present invention is intended for use in the treatment of peripheral artery disease (PAD), of critical limb ischemia (CLI) and of diabetic foot.

The autologous preparation (A) of the present invention is obtained by dilution, in autologous platelet-enriched plasma (B) or in platelet concentrate (B'), of an autologous concentrate (C) comprising peripheral blood mononuclear cells (PNMNCs), wherein said autologous concentrate (C) is free of red blood cells, neutrophil-depleted and comprises Dimethylsulfoxide (DMSO) in concentrations comprised between 3% and 5% in volume on the volume of the autologous concentrate (C).

In a particularly preferred embodiment, the autologous preparation (A) is obtained by dilution of said autologous concentrate (C), further comprising albumin.

Even more preferably, the autologous concentrate comprises the peripheral blood mononuclear cells (PBMNCs):
- Total nucleated cells (TNCs), comprised between 10.5 × 10³/µL and 13.6 × 10³/µL,
- Lymphocytes, comprised between 5.0 × 10³/µL and 7.0 × 10³/µL,
- Monocytes, comprised between 1.0 × 10³/µL and 2.0 × 10³/µL,
- Mononuclear cells (MNCs), comprised between 6.2 × 10³/µL and 9.3 × 10³/µL.

The autologous concentrate (C) according to the present invention preferably comprises a neutrophil concentration which varies from 0.1 × 10³/µL and 0.2 × 10³/µL.

As already explained, the autologous preparation (A) object of the present invention is obtained by a process which comprises in particular the following steps:
i) obtaining, by treating a blood sample taken from the patient, autologous PBMNCs and platelet-enriched plasma (B) or platelet concentrate (B'), respectively;
ii) adding DMSO to the PBMNCs obtained at step (i), at a concentration comprised between 3% and 5% (v/v) dissolved in a portion of platelet-enriched plasma (B) or platelet concentrate (B') prepared in step i) and obtaining the autologous concentrate (C);
iii) freezing the autologous concentrate (C) of item ii) and the autologous platelet-enriched plasma (B) or platelet concentrate (B') as obtained at step i);
iv) sending the autologous concentrate (C) and the autologous platelet-enriched plasma (B) or platelet concentrate (B') of step iii) to the hospital site of use;
v) thawing upon use the autologous concentrate (C) and the autologous platelet-enriched plasma (B) or platelet concentrate (B') of item iv),
vi) diluting from 7 to 10 times, preferably 8 times, the volume of the thawed autologous concentrate (C), with autologous plasma (B) or thawed platelet concentrate (B') and obtaining the autologous preparation (A).

It should be noted that said diluting step vi) advantageously allows to dilute the DMSO and at the same time to obtain the effective concentrations of the peripheral blood mononuclear cells contained in the autologous preparation (A).

The DMSO dilution advantageously allows eliminating the toxicity in vivo at the moment of the implantation.

According to a preferred embodiment, the step of diluting vi) the thawed autologous concentrate (C), with the autologous plasma (B) or platelet concentrate (B'), is the only diluting step of the process for preparing the autologous preparation (A) to reduce the DMSO concentrations in the autologous preparation (A).

Preferably, the process for preparing the autologous preparation (A) object of the present application does not comprise any rinsing step for reducing the DMSO concentrations in the autologous preparation (A).

The preparation (A) is therefore applied to the patient in need of such a treatment.

In a preferred embodiment, step ii) provides the further addition of albumin to PBMNCs.

In a particularly preferred embodiment, the process for preparing the autologous preparation (A), object of the present invention, provides that the autologous concentrate (C) obtained in point ii), is preferably preconditioned in hypoxia, at an O₂ concentration before the freezing step of point iii).

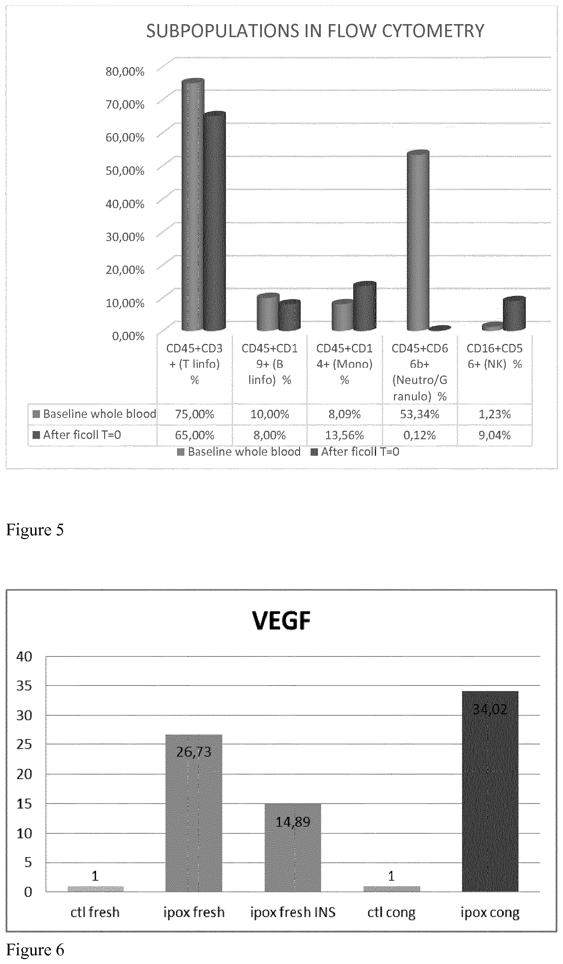

Such hypoxic preconditioning allows considerably increasing the therapeutic angiogenic capacity of the preparation in the ischemic tissues, and in particular the release of important angiogenic factors such as, for example the VEGF (Fig. 6).

The experimental data shown in Fig. 6 show how the fresh (not frozen) autologous concentrate, preconditioned with hypoxia (ipox fresh), is characterised by an increased release of VEGF (26.73%) with respect to the control (ctl fresh), consisting of non-treated fresh autologous concentrate (1%).

Even more interestingly, the release of angiogenic factors, induced by the hypoxic preconditioning, remains even after freezing (Fig. 6): the hypoxic preconditioned and frozen autologous concentrate (ipox cong) is characterised by an increase release of angiogenic factors (34.02%) with respect to the frozen hypoxic preconditioned fresh autologous concentrate (ipox fresh = 26.73%).

Step i) or the preparation of PBMNCs and of the autologous plasma (B) or of the platelet concentrate (B') is preferably obtained through a process which comprises the following steps:
I. 1:1 volumetric dilution of blood taken from the patient in a biologically compatible buffer and in presence of an anticoagulant.
II. Loading, in a 1:1 volumetric ratio, inside tubes with inner septum, blood coming from step (I) and a Ficoll density gradient polymer solution;
III. Centrifuging the tubes of step (II) at 1200 x g for 10 minutes, at room temperature and separating the supernatant comprising mononuclear cells (PBMNCs) from autologous platelet-enriched plasma (B).
IV. Washing the PBMNCs coming from step (III) twice, with a biologically-compatible buffer system, by centrifuging at 300 x g for 8 minutes.
V. Optionally concentrating autologous plasma (B) by centrifugation and separation of the heavier fraction and obtaining platelet concentrate (B').

In step (ii) the autologous concentration (C) is prepared by diluting the PBMNCs coming from step (i), with a specific volumetric portion, preferably comprised between 8-12 cm³, more preferably of 10 cm³ of autologous plasma (B), of item III, comprising from 3% to 5% of DMSO and from 1% to 5% of albumin.

The freezing or step (iii) of the process object of the present invention is preferably carried out at -80°C.

The autologous concentrate thus obtained, once frozen may be stored at the same temperature for a maximum period of 2 years.

In another embodiment, the freezing and the storing of concentrate are carried out preferably through controlled temperature reduction in tanks of liquid nitrogen vapours, where the composite reaches the temperature of -140° in 1 hour of time. In this case the composite may be stored for a maximum period of 5 years.

For an illustrative and non-limiting purpose, an example relative to the autologous preparation according to the present invention is given.

### EXAMPLE 1

In this example in the following table 1, column 4 the absolute cellular count values of the autologous preparation object of the present invention are reported, obtained with the autologous concentration in PBMNCs of column 2 in table 1.

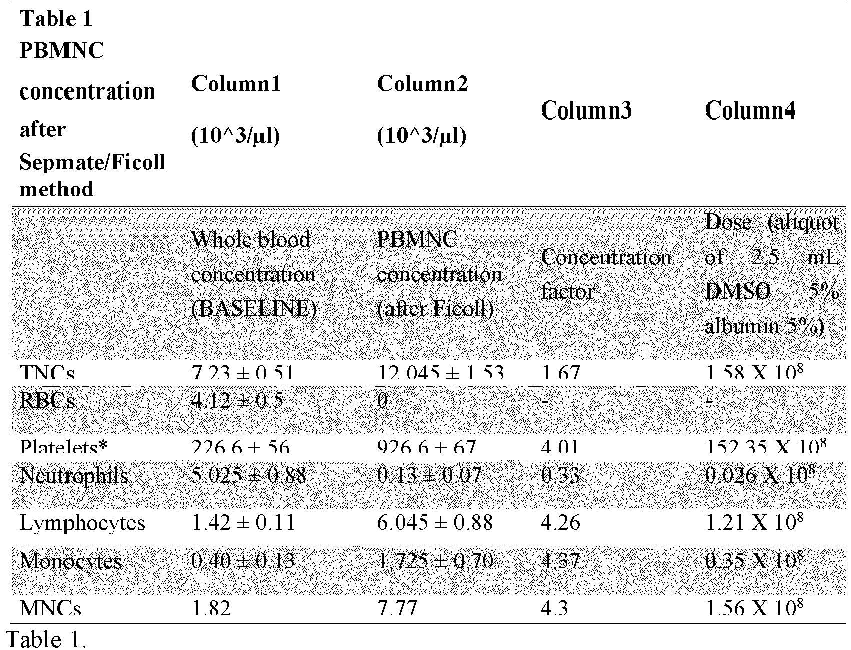

The method for producing the autologous preparation according to the present invention, comprising the following steps of:
From a single blood sample (200 mL), obtaining two concentrate aliquots of 2.5 mL each (5 mL in total), comprising each PBMNCs in the concentrations listed in column 2 of Table 1.

Each aliquot of 2.5 mL corresponding to a dose of autologous preparation to be implanted.

For each 5 mL of autologous concentrate of mononuclear cells, obtaining 20 mL of autologous platelet-enriched plasma, starting from a same blood sample (200 mL).

The treatment described above allows isolating the PBMNCs from the autologous plasma, eliminating the risk of contamination from red blood cells and reducing the neutrophil content.

Said method of isolating the peripheral blood mononuclear cells results in a high standardisation of the product, both as regards the fraction of peripheral blood mononuclear cells, and as regards the protein-enriched plasma fraction.

The concentrate and the plasma thus obtained are then frozen as in item iv) described above and stored as such until necessary.

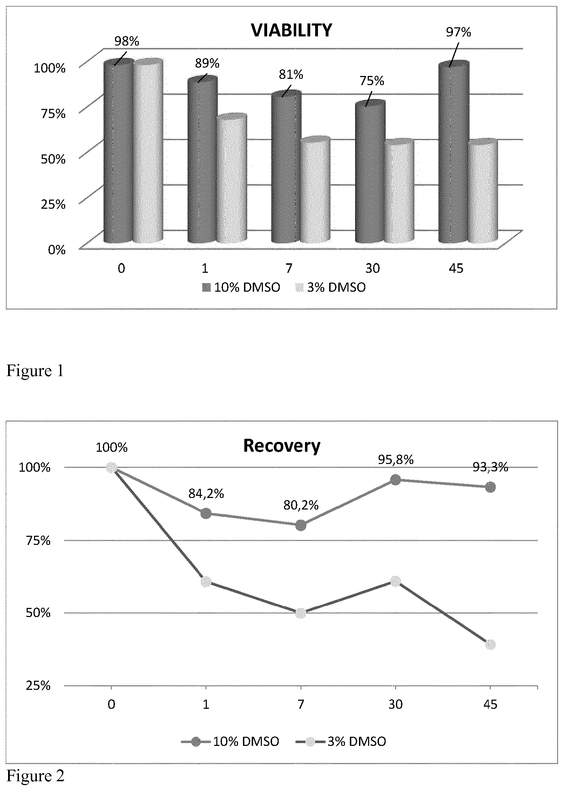

Despite the low DMSO concentrations, generally used at concentrations of about 10% in the cellular storage techniques, the autologous concentrate of the present invention maintains a high cellular viability over time, as demonstrated by the experimental data in Figures 1, 2 and 3.

Two tests were carried out with different concentrations of DMSO, at 3% and at 10% respectively, measuring the viability of the concentrate after thawing, and before forming the preparation by suspension.

The viability was measured at the moment of preparation (t = 0), after 1, 7, 30 and 45 days from preparation (t = 1, t = 7, t = 30, t = 45)

The viability data reported in Figure 1 show how the DMSO better stores the cells over time, with a particularly high viability value (97%) after 45 days from thawing the autologous concentrate.

Similarly to the measurement of the viability, the cellular yield of the thawed autologous concentrate was measured, when prepared with DMSO at 3% and at 10%,

The data thus obtained, reported in figure 2, show a better yield for the concentrate comprising the highest concentrations of DMSO (10%), with a percentage value of 93.3% after 45 days from thawing the cellular substrate.

The autologous concentrate prepared with DMSO at 3% stores an optimal yield until the day of thawing (100%), with halving only at the seventh day after thawing, thus in any case indicating a product of high quality, to be immediately used.

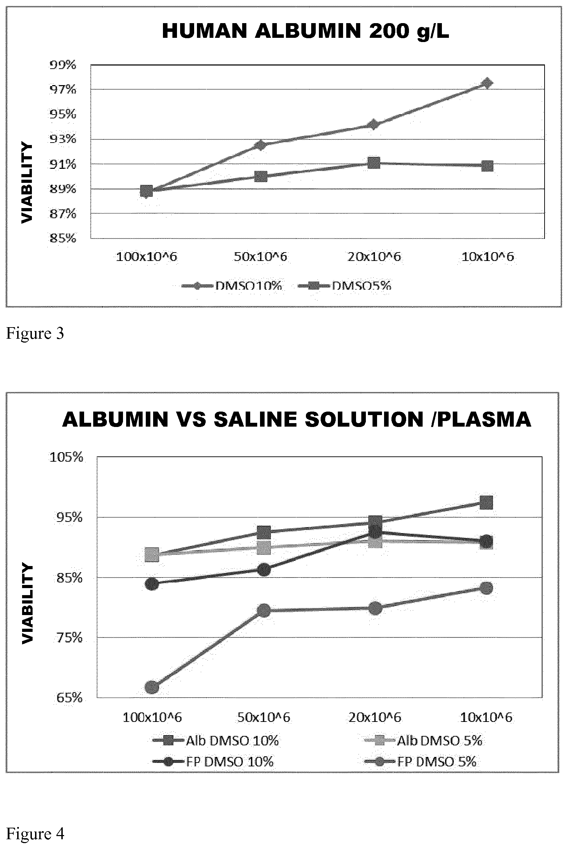

A further improvement of the viability is obtained freezing in plasma, albumin 5% and DMSO 5% (Fig. 4).

The thawing at the moment of use provides that each aliquot of 2.5 mL of concentrate is diluted in 20 mL of autologous plasma, according to the method of producing the autologous preparation described above.

Suspending the autologous concentrate in plasma allows diluting DMSO up to concentrations comprised between 0.3% and 1.3%, thus eliminating the toxicity risk at the moment of implantation.

At the same time of diluting the cryopreservative, the suspension in plasma allows to obtain the cellular concentrations suitable for the administration and for the exhibition of the therapeutic effect.

The advantages which the autologous preparation of the present invention has with respect to the autologous preparations known to the prior art may be thus summarised:
- High standardisation and reproducibility responding to the guidelines established by the European Medicines Agency for producing medicinal specialities which fall under the Advanced Therapies regulation (ATMPs - European Regulation 1394/2007);
- Optimisation of the blood sample to be treated and obtaining from the same two aliquots of autologous concentrate, as well as platelet-enriched plasma;
- Freezable and thawable product, which allows an easy management of the biological material, to be thawed and restored at the moment of use;
- Easy storing in frozen form, for long times;
- Maintaining a good viability over time, even after thawing;
- Amplification of the angiogenic effect of the cells through hypoxic preconditioning.

## Claims

1. An autologous preparation (A) substantially red blood cell-free and neutrophil-depleted, comprising:
a) Peripheral blood mononuclear cells (PBMNCs),
b) Dimethylsulfoxide (DMSO) in concentrations comprised between 0.3% and 1.3% (v/v),
c) Autologous platelet-enriched plasma (B), having a platelet concentration comprised between 700,000 platelets/µL and 1,200,000 platelets/µL; or platelet concentrate (B'), having a platelet concentration comprised between 7,000,000 platelets/µL and 12,000,000 platelets/µL,
said autologous preparation (A) being obtained with a process comprising the following steps:
i) Obtaining, by treating a blood sample taken from the patient, autologous PBMNCs and platelet-enriched plasma (B) or platelet concentrate (B'), respectively;
ii) Adding DMSO to the PBMNCs obtained at step (i), at a concentration comprised between 3% and 5% (v/v) dissolved in a portion of platelet-enriched plasma (B) or platelet concentrate (B') prepared in step i) and obtaining an autologous concentrate (C);
iii) Freezing the autologous concentrate (C) of item ii) and the autologous platelet-enriched plasma (B) or platelet concentrate (B') as obtained at step i);
iv) Sending the autologous concentrate (C) and the autologous platelet-enriched plasma (B) or platelet concentrate (B') of step iii) to the desired health care centre;
v) Thawing, upon use, the autologous concentrate (C) and the autologous platelet-enriched plasma (B) or platelet concentrate (B') of item iv),
vi) Diluting, 7 to 10 times, the volume of thawed autologous concentrate (C) with thawed autologous plasma (B) or thawed platelet concentrate (B') and obtaining the autologous preparation (A),
wherein said autologous concentrate (C) comprises mononuclear cells (PBMNCs), is red blood cell-free, neutrophil-depleted and comprises dimethylsulfoxide (DMSO) at a concentration comprised between 3vol% and 5vol% based on the volume of autologous concentrate (C).

2. Autologous preparation (A) according to claim 1, containing albumin.

3. Autologous preparation (A) according to claim 1 or 2, wherein said peripheral blood mononuclear cells (PBMNCs) comprise:
- TNCs comprised between 1.26 × 10⁸ and 1.89 × 10⁸ (6.3 × 10³ and 9,45 × 10³ cells/µL),
- Lymphocytes comprised between 0.97 × 10⁸ and 1.45 × 10⁸ (4.85 × 10³ and 7,25 × 10³ cells/µL),
- Monocytes comprised between 0.28 × 10⁸ and 0.42 × 10⁸ (1.4 × 10³ and 2,1 × 10³ cells/µL),
- MNCs comprised between 1.25 × 10⁸ and 1.87 × 10⁸ (6.25 × 10³ and 9,35 × 10³ cells/µL).

4. Autologous preparation (A) according to any one of claims from 1 to 3, wherein the neutrophil content is comprised between 0.015 × 10⁸ and 0.029 × 10⁸ (75 and 145 cells/µL).

5. Autologous preparation (A) according to any one of claims 1 to 4 for use as a medicament.

6. Autologous preparation (A) for use according to claim 5, for the treatment of peripheral artery disease (PAD), critical limb ischemia (CLI), and diabetic foot.

7. Autologous preparation (A) according to claim 1, wherein said autologous concentrate (C) comprises albumin.

8. Autologous preparation (A) according to claim 1, wherein said autologous concentrate (C) comprises:
- Total nucleated cells (TNCs) comprised between 10.5 × 10³/µL and 13.6 × 10³/µL,
- Lymphocytes comprised between 5.0 × 10³/µL and 7.0 × 10³/µL,
- Monocytes comprised between 1.0 × 10³/µL and 2.0 × 10³/µL,
- Mononuclear cells (MNCs) comprised between 6.2 × 10³/µL and 9.3 × 10³/µL,

9. Autologous preparation (A) according to claim 1, wherein the neutrophil content in said autologous concentrate (C) is comprised between 0.1 × 10³ and 0.2 × 10³/µL.

10. Autologous preparation (A) according to anyone of claims 1-9, wherein step i), of preparing the PBMNC cells and plasma (B) or platelet concentrate (B'), is achieved by a process comprising the following steps:
I. 1:1 volumetric dilution of blood taken from the patient in a biologically-compatible buffer and in the presence of an anticoagulant;
II. Loading, in a 1:1 volumetric ratio, inside tubes with inner septum, blood coming from step (I) and a Ficoll density gradient polymer solution;
III. Centrifuging the tubes of step (II) at 1200 x g for 10 minutes, at room temperature and separating the supernatant comprising mononuclear cells (PBMNCs) from autologous platelet-enriched plasma (B).
IV. Washing the PBMNCs coming from step (III) twice, with a biologically-compatible buffer system, by centrifuging at 300 x g for 8 minutes.
V. Optionally concentrating autologous plasma (B) by centrifugation and separation of the heavier fraction and obtaining platelet concentrate (B').

11. Autologous preparation (A) according to claim 1, wherein in step ii) albumin is added to PBMNCs.

12. Autologous preparation (A) according to anyone of claims 1-11, wherein the autologous concentrate (C) obtained at item ii) is pre-conditioned prior to the freezing step of item iii).

13. Autologous preparation (A) according to anyone of claims 1-12, , wherein the freezing step (iii) is carried out at -80°C.

14. Autologous preparation (A) according to anyone of claims 1-13, wherein in step vi) the initial volume of the autologous concentrate (C) is diluted up to 8 times in platelet-enriched plasma (B) or platelet concentrate (B').

## Patentansprüche

1. Autologe Zubereitung (A), das im Wesentlichen frei von roten Blutkörperchen und abgereichert an Neutrophilen ist, umfassend:
a) Mononukleare Zellen aus peripherem Blut (PBMNCs),
b) Dimethylsulfoxid (DMSO) in Konzentrationen zwischen 0,3% und 1,3% (v/v),
c) Autologes, mit Blutplättchen angereichertes Plasma (B), das eine Blutplättchenkonzentration zwischen 700.000 Blutplättchen/µL und 1.200.000 Blutplättchen/µL aufweist; oder Blutplättchenkonzentrat (B'), das eine Blutplättchenkonzentration zwischen 7.000.000 Blutplättchen/µL und 12.000.000 Blutplättchen/µL aufweist,
wobei die Autologe Zubereitung (A) mit einem Verfahren erhalten wird, das die folgenden Stufen umfasst:
i) Gewinnung von autologen PBMNCs und mit Blutplättchen angereichertem Plasma (B) bzw. Blutplättchenkonzentrat (B') durch Behandlung einer vom Patienten eingenommenen Blutprobe;
ii) Zugabe von DMSO zu den in Stufe (i) erhaltenen PBMNCs in einer Konzentration zwischen 3 % und 5 % (v/v), gelöst in einem Teil des mit Blutplättchen angereicherten Plasmas (B) oder des in Stufe (i) hergestellten Blutplättchenkonzentrats (B'), und Gewinnung eines autologen Konzentrats (C);
iii) Einfrieren des autologen Konzentrats (C) von Punkt ii) und des autologen, mit Blutplättchen angereicherten Plasmas (B) oder Blutplättchenkonzentrats (B'), wie in Stufe i) erhalten;
iv) Versenden des autologen Konzentrats (C) und des autologen, mit Blutplättchen angereicherten Plasmas (B) oder Blutplättchenkonzentrats (B') der Stufe iii) an das gewünschte Gesundheitszentrum;
v) Auftauen des autologen Konzentrats (C) und des autologen, mit Blutplättchen angereicherten Plasmas (B) oder Blutplättchenkonzentrats (B') von Punkt iv) anhand der Verwendung,
vi) Abschwächen des 7- bis 10-fachen Volumens des aufgetauten autologen Konzentrats (C) mit aufgetautem autologen Plasma (B) oder aufgetautem Blutplättchenkonzentrat (B') und Gewinnen der autologen Zubereitung (A),
wobei das autologe Konzentrat (C) mononukleare Zellen (PBMNCs) umfasst, frei von roten Blutkörperchen und abgereichert an Neutrophilen ist und Dimethylsulfoxid (DMSO) in einer Konzentration zwischen 3 Vol% und 5 Vol%, bezogen auf das Volumen des autologen Konzentrats (C), umfasst.

2. Autologe Zubereitung (A) nach Anspruch 1, das Albumin enthält.

3. Autologe Zubereitung (A) nach Anspruch 1 oder 2, wobei die mononukleären Zellen des peripheren Blutes (PBMNCs) umfassen:
- TNCs, die zwischen 1,26 × 10⁸ und 1,89 × 10⁸ (6,3 × 10³ und 9,45 × 10³ Zellen/µL) enthalten sind,
- Lymphozyten, die zwischen 0,97 × 10⁸ und 1,45 × 10⁸ (4,85 × 10³ und 7,25 × 10³ Zellen/µL) enthalten sind,
- Monozyten, die zwischen 0,28 × 10⁸ und 0,42 × 10⁸ (1,4 × 10³ und 2,1 × 10³ Zellen/µl) enthalten sind,
- MNCs die zwischen 1,25 × 10⁸ und 1,87 × 10⁸ (6,25 × 10³ und 9,35 × 10³ Zellen/µl) enthalten sind.

4. Autologe Zubereitung (A) nach einem der Ansprüche 1 bis 3, wobei der Inhalt an Neutrophilen zwischen 0,015 × 10⁸ und 0,029 × 10⁸ (75 und 145 Zellen/µL) liegt.

5. Autologe Zubereitung (A) nach einem der Ansprüche 1 bis 4 zur Verwendung als Arzneimittel.

6. Autologe Zubereitung (A) zur Verwendung gemäß Anspruch 5 für die Behandlung der peripheren Arterienerkrankung (PAD), der kritischen Extremitätenischämie (CLI) und des diabetischen Fußes.

7. Autologe Zubereitung (A) nach Anspruch 1, wobei das autologe Konzentrat (C) Albumin umfasst.

8. Autologe Zubereitung (A) nach Anspruch 1, wobei das autologe Konzentrat (C) folgendes umfasst:
- Nukleierte Gesamtzellen (TNCs), die zwischen 10,5 × 10³/µL und 13,6 × 10³/µL enthalten sind,
- Lymphozyten, die zwischen 5,0 × 10³/µl und 7,0 × 10³/µl enthalten sind,
- Monozyten, die zwischen 1,0 × 10³/µl und 2,0 × 10³/µl enthalten sind,
- Mononukleäre Zellen (MNCs) umfassten zwischen 6,2 × 10³/µL und 9,3 × 10³/µL.

9. Autologe Zubereitung (A) nach Anspruch 1, wobei der Gehalt an Neutrophilen in dem autologen Konzentrat (C) die zwischen 0,1 × 10³ und 0,2 × 10³/µL enthalten sind.

10. Autologe Zubereitung (A) nach einem der Ansprüche 1 bis 9, wobei die Stufe i) der Herstellung der PBMNC-Zellen und des Plasmas (B) oder Blutplättchenkonzentrats (B') durch ein Verfahren erreicht wird, das die folgenden Stufen umfasst:
I. Volumetrische 1:1-Verdünnung von Blut, das dem Patienten in einem biologisch kompatiblen Puffer und in Gegenwart eines Antikoagulans abgenommen wurde;
II. Einfüllen des aus Stufe (I) stammenden Blutes und einer Ficoll-Dichtegradienten-Polymerlösung in Röhrchen mit innerem Septum in einem volumetrischen Verhältnis von 1:1;
III. Zentrifugieren der Röhrchen aus Stufe (II) bei 1200 x g für 10 Minuten bei Raumtemperatur und Trennen des Überstands, der mononukleäre Zellen (PBMNCs) enthält, von autologem, mit Blutplättchen angereichertem Plasma (B).
IV. Zweimaliges Waschen der aus Stufe (III) stammenden PBMNCs mit einem biologisch verträglichen Puffersystem durch Zentrifugieren bei 300 x g für 8 Minuten.
V. Optionales Konzentrieren des autologen Plasmas (B) durch Zentrifugation und Abtrennung der schwereren Fraktion und Gewinnung des Blutplättchenkonzentrats (B').

11. Autologe Zubereitung (A) nach Anspruch 1, wobei in Stufe ii) Albumin zu den PBMNCs hinzugefügt wird.

12. Autologe Zubereitung (A) nach einem der Ansprüche 1-11, wobei das in Punkt ii) erhaltene autologe Konzentrat (C) vor der Stufe des Einfrierens in Punkt iii) vorkonditioniert wird.

13. Autologe Zubereitung (A) nach einem der Ansprüche 1-12, wobei die Stufe des Einfrierens (iii) bei -80°C durchgeführt wird.

14. Autologe Zubereitung (A) nach einem der Ansprüche 1-13, wobei in Stufe vi) das Ausgangsvolumen des autologen Konzentrats (C) bis zum 8-fachen in mit Blutplättchen angereichertem Plasma (B) oder Blutplättchenkonzentrat (B') abgeschwächt wird.

## Revendications

1. Préparation autologue (A) sensiblement exempte de globules rouges et appauvrie en neutrophiles, comprenant:
a) Des cellules mononucléées du sang périphérique (PBMC),
b) Diméthylsulfoxyde (DMSO) à des concentrations comprises entre 0,3 % et 1,3 % (v/v),
c) Plasma riche en plaquettes autologue (B), ayant une concentration en plaquettes comprise entre 700.000 plaquettes/µL et 1.200.000 plaquettes/µL ; ou un concentré de plaquettes (B'), ayant une concentration en plaquettes comprise entre 7.000.000 plaquettes/µL et 12.000.000 plaquettes/µL,
ladite préparation autologue (A) étant obtenue par un procédé comprenant les étapes suivantes :
i) Obtenir, par traitement d'un échantillon de sang prélevé sur le patient, respectivement, des PBMC autologues et du plasma riche en plaquettes (B) ou du concentré de plaquettes (B');
ii) Ajouter du DMSO aux PBMC obtenues à l'étape (i), à une concentration comprise entre 3% et 5% (v/v) dissous dans une portion de plasma riche en plaquettes (B) ou de concentré de plaquettes (B') préparé à l'étape (i) et obtenir un concentré autologue (C) ;
iii) Congeler le concentré autologue (C) du point ii) et le plasma riche en plaquettes autologue (B) ou le concentré de plaquettes (B') tel qu'obtenu à l'étape i);
iv) Envoyer le concentré autologue (C) et le plasma riche en plaquettes autologue (B) ou le concentré de plaquettes (B') de l'étape iii) au centre de santé souhaité ;
v) Dégeler, lors de l'utilisation, le concentré autologue (C) et le plasma riche en plaquettes autologue (B) ou le concentré de plaquettes (B') du point iv),
vi) Diluer, 7 à 10 fois, le volume du concentré autologue dégelé (C) avec du plasma autologue dégelé (B) ou du concentré de plaquettes dégelé (B') et obtenir la préparation autologue (A),
dans laquelle ledit concentré autologue (C) comprend des cellules mononucléées (PBMC), est exempt de globules rouges, appauvri en neutrophiles et comprend du diméthylsulfoxyde (DMSO) à une concentration comprise entre 3% en volume et 5% en volume sur la base du volume de concentré autologue (C).

2. Préparation autologue (A) selon la revendication 1, contenant de l'albumine.

3. Préparation autologue (A) selon la revendication 1 ou 2, dans laquelle lesdites cellules mononucléées du sang périphérique (PBMC) comprennent:
- CNT comprenant entre 1,26 × 10⁸ et 1,89 × 10⁸ (6.3 × 10³ et 9,45 × 10³ cellules/µL),
- Lymphocytes compris entre 0.97 × 10⁸ et 1.45 × 10⁸ (4.85 × 10³ et 7,25 × 10³ cellules/µL),
- Monocytes compris entre 0.28 × 10⁸ et 0.42 × 10⁸ (1.4 × 10³ et 2,1 × 10³ cellules/µL),
- CMN compris entre 1.25 × 10⁸ et 1.87 × 10⁸ (6,25 × 10³ et 9,35 × 10³ cellules/µL).

4. Préparation autologue (A) selon l'une quelconque des revendications 1 à 3, dans laquelle la teneur en neutrophiles est comprise entre 0.015 × 10⁸ et 0.029 × 10⁸ (75 et 145 cellules/µL).

5. Préparation autologue (A) selon l'une quelconque des revendications 1 à 4, destinée à être utilisée comme médicament.

6. Préparation autologue (A) destinée à l'utilisation selon la revendication 5, pour le traitement de la maladie artérielle périphérique (MAP), de l'ischémie critique des membres inférieurs (ICMI) et du pied diabétique.

7. Préparation autologue (A) selon la revendication 1, dans laquelle ledit concentré autologue (C) comprend de l'albumine.

8. Préparation autologue (A) selon la revendication 1, dans laquelle ledit concentré autologue (C) comprend :
- Des cellules nucléées totales (CNT) comprises entre 10.5 × 10³/µL et 13.6 × 10³/µL,
- Des lymphocytes compris entre 5.0 × 10³/µl et 7.0 × 10³/µL,
- Des monocytes compris entre 1.0 × 10³/µL et 2.0 × 10³/µL,
- Des cellules mononucléées (CMN) comprises entre 6.2 × 10³/µL et 9.3 × 10³/µL.

9. Préparation autologue (A) selon la revendication 1, dans laquelle la teneur en neutrophiles dans ledit concentré autologue (C) est comprise entre 0.1 × 10³ et 0.2 × 10³/µL.

10. Préparation autologue (A) selon l'une quelconque des revendications 1 à 9, dans laquelle l'étape i) de préparation des cellules PBMC et du plasma (B) ou du concentré de plaquettes (B') est réalisée par un procédé comprenant les étapes suivantes :
I. Dilution volumétrique 1:1 du sang prélevé sur le patient dans un tampon biologiquement compatible et en présence d'un anticoagulant;
II. Chargement, dans un rapport volumétrique de 1:1, à l'intérieur des tubes avec un septum interne, du sang provenant de l'étape (I) et d'une solution de polymères Ficoll par gradient de densité ;
III. Centrifugation des tubes de l'étape (II) à 1200 x g pendant 10 minutes, à température ambiante et séparation du surnageant comprenant des cellules mononucléées (PBMC) du plasma riche en plaquettes autologue (B).
IV. Lavage des PBMC issus de l'étape (III) deux fois, avec un système tampon biologiquement compatible, par centrifugation à 300 x g pendant 8 minutes.
V. Concentration éventuelle du plasma autologue (B) par centrifugation et séparation de la fraction la plus lourde et obtention du concentré de plaquettes (B').

11. Préparation autologue (A) selon la revendication 1, dans laquelle, dans l'étape ii), de l'albumine est ajoutée aux PBMC.

12. Préparation autologue (A) selon l'une quelconque des revendications 1 à 11, dans laquelle le concentré autologue (C) obtenu au point ii) est préconditionné avant l'étape de congélation du point iii).

13. Préparation autologue (A) selon l'une quelconque des revendications 1 à 12, dans laquelle l'étape de congélation (iii) est effectuée à -80°C.

14. Préparation autologue (A) selon l'une quelconque des revendications 1 à 13, dans laquelle, dans l'étape vi), le volume initial du concentré autologue (C) est dilué jusqu'à 8 fois dans du plasma riche en plaquettes (B) ou du concentré de plaquettes (B').
